# EUROPÄISCHE PATENTSCHRIFT

(11) **EP 2 338 559 B1**
(45) Veröffentlichungstag und Bekanntmachung des Hinweises auf die Patenterteilung: **04.03.2015**
(21) Anmeldenummer: 10192901.6
(22) Anmeldetag: 29.11.2010
(51) Int. Cl.: A61N 1/08, A61N 1/37, A61N 1/39

(54) **MRI-Gradientenfelddetektor**
MRI gradient field detector
Détecteur de champ à gradients IRM

(30) Priorität: 22.12.2009 US 288855 P
(43) Veröffentlichungstag der Anmeldung: 29.06.2011
(73) Patentinhaber: BIOTRONIK CRM Patent AG, 6341 Baar (CH)
(72) Erfinder: Dörr, Thomas, 12437, Berlin (DE); Weiss, Ingo, 12435, Berlin (DE)
(74) Vertreter: Lindner-Vogt, Karin L.

(56) Entgegenhaltungen:
- EP-A1- 1 716 878
- EP-A1- 1 935 450
- WO-A1-96/41203
- US-A1- 2004 088 012
- US-A1- 2006 293 591

## Beschreibung

Die Erfindung bezieht sich auf eine Vorrichtung und ein Verfahren zur Erkennung von elektromagnetischen Feldern, speziell solche Felder, wie sie bei bildgebenden Kernspintomographien- (im folgenden MRT oder MRI) Geräten auftreten.

Obwohl MRI-Untersuchungen einen immer höheren Stellenwert in der diagnostischen Medizin erhalten, ist ein Teil der Patienten für MRI-Untersuchungen kontraindiziert. Eine solche Kontraindikation kann durch ein aktives implantiertes medizinisches Gerät (im Folgenden auch Implantat oder IMD) gegeben sein. Neben den MRI-Untersuchungen stellen aber auch andere technische Anwendungen eine Gefahr für die Nutzer von medizinischen Geräten oder implantierbaren medizinischen Geräten dar, besonders wenn diese starke elektromagnetische Störfelder (EMI, Electro Magnetic Interference) in Ihrer Umgebung erzeugen.

Um MRI-Untersuchungen dennoch zu ermöglichen, sind verschiedene Ansätze, die sich entweder auf die Durchführung der MRI-Untersuchung oder auf das implantierbare medizinische Gerät beziehen, bekannt.

Unter anderem sind Technologien zur Erkennung von Magnetfeldern bekannt, die auf herkömmlichen Verfahren zur Magnetfelddetektion beruhen. So beschreibt die US 2008/0154342 ein Verfahren unter Ausnutzung eines GMR-Sensors (Giant Magnetic Resistance), um problematische Magnetfelder von MRT-Geräten zu erkennen.

Des Weiteren existiert auch ein Ansatz in der US 2006/0293591A1, der darauf abzielt eine MRI-Erkennung auf Basis der Auswertung von Spannungen an der Kommunikationsspule und einer Elektrode vorzunehmen. Diese Lösung hat jedoch den Nachteil, dass die reine Spannungsbewertung für die Erkennung eines Gradientenfeldes sehr unspezifisch ist. Die an der Programmierspule (240) durch das Gradientenfeld induzierten Spannungen sind extrem variabel. Gleiches gilt für die Spannungen an der Elektrode, typischerweise induziert durch das RF-Feld und das Gradientenfeld. Außerdem sind hier zusätzliche Spannungswandler nötig, die die Spannungsmessung zeitgleich an der Kommunikationsspule und mindestens einer Elektrode ermöglichen. Außerdem existieren Konfigurationen in Bezug auf die Lage des Magnetfeldes relativ zur Implantatslage, die eine Erkennung des Gradientenfeldes verhindern.

Ein weiterer Ansatz auf der Basis der an der Programmierspule induzierten Spannungen ist aus der EP 1 935 450 A1 bekannt.

Daher ist es Aufgabe der vorliegenden Erfindung, eine einfache und zuverlässige Vorrichtung und Methode zur Erkennung von typischen MRT-Feldern für ein IMD bereitzustellen, die die Nachteile des Standes der Technik behebt.

Die Aufgabe wird von einem implantierbaren medizinischen Gerät (IMD) nach Anspruch 1 und ein Verfahren nach Anspruch 11 gelöst.

Das IMD umfasst dabei mindestens:
mit einem hermetisch abgeschlossenem Gehäuse, mindestens einer Steuereinheit, mindestens einer Programmierspule (240), mindestens einer Kommunikationseinheit und einer Erkennungseinheit für MRT-Störfelder (250), wobei die Kommunikationseinheit im Zusammenwirken mit der Programmierspule (240) ausgestaltet ist, eine Kommunikation zwischen einem externen Programmiergerät und dem implantierten medizinischen Gerät (200) unter Nutzung von elektromagnetischen Wechselfeldern zu ermöglichen, wobei die Erkennungseinheit für MRT-Störfelder (250) derart ausgebildet ist, dass von einem gepulsten elektromagnetischen Wechselfeld des MRT herrührende in die Programmierspule (240) induzierte Spannungen oder Spannungsverläufe erkannt werden und ein MRT-Erkennungssignal von der Erkennungseinheit für MRT-Störfelder (250) an die mindestens eine Steuereinheit übermittelt wird, sofern nicht zeitgleich eine Kommunikation mit einem externen Programmiergerät über die Programmierspule erkannt wird.

Die gepulsten elektromagnetischen Wechselfelder können insbesondere Gradientenfelder sein.

Vorteilhaft bei der Erkennung von gepulsten elektromagnetischen Wechselfeldern ist, dass einerseits das Erkennen von MRI typischen Störfeldern erleichtert wird und auf der anderen Seite gleichzeitig ein so genanntes "Fehlsensing" vermieden wird. Unter "Fehlsensing" ist in diesem Zusammenhang zu verstehen, dass ein IMD eine gepulste magnetische Störung als ein Signal aus dem Herzen interpretiert wird und dadurch keine korrekten Schlussfolgerungen auf den Gesundheitszustand des Patienten möglich sind. Solche "Fehlsensings" werden durch die explizite Erkennung von gepulsten elektromagnetischen Störungen vermieden. Auch erlaubt die Erkennung der gepulsten MRI-Gradientenfelder eine Unterscheidung der MRI bedingten Störungen von anderen elektromagnetischen Störungen.

Bevorzugt ist, dass die Erkennungseinheit für MRT-Störfelder (250) die gepulsten elektromagnetischen Wechselfelder durch eine Schwellwertdetektion und eine Zählung der induzierten Spannungspulse pro Zeiteinheit erkennt.

Ebenfalls bevorzugt ist, dass die MRT-Störerkennung auf Auswertung und Vergleich der induzierten Spannungen oder des Spektrums der induzierten Spannungen mit MRT-typischen induzierten Spannungen oder MRT-typischen induzierten Spannungsspektren beruht.

Auch wird bevorzugt, dass das IMD zusätzlich mit mindestens einer Elektrode verbunden ist, die mit der Erkennungseinheit für MRT-Störfelder (250) verbunden ist und ein MRT-Erkennungssignal nur dann übermittelt wird, wenn die Störung zeitgleich sowohl über die Programmierspule (240), als auch über mindestens eine Elektrode erkannt wird.

Eine weitere bevorzugte Ausfiihrungsform ist, dass das MRT-Erkennungssignal einen Wechsel des Betriebszustands des IMD bewirkt, bevorzugt in einen MRT-sicheren Zustand, wobei dieser Zustand entweder bis zu einer möglichen Umprogrammierung permanent oder für einen vorbestimmten Zeitraum befristet oder bis zu einem Ausbleiben einer MRT-Erkennung oder bis zu einem Ausbleiben einer MRT-Erkennung für einen vorbestimmten Zeitraum beibehalten wird.

Auch wird bevorzugt, dass die Gradientenfelderkennung über einen programmierbaren Filter und eine Triggereinheit erfolgt, die mit der Programmierspule (240) verbunden sind, so dass über die Programmierung des Filters die Gradientenfelderkennung an unterschiedliche MRT-Anlagen angepasst und/oder automatisch angepasst werden kann.

Ebenfalls bevorzugt wird, dass ein MRT-Zustand permanent, bis zur nächsten Umprogrammierung, oder für eine vorbestimmte erste Zeit oder eine einstellbare erste Zeit einstellbar ist, bei dem die VF-Detektion um eine vorbestimmte zweite Zeit verlängert wird, um eine Gradientenfelderkennung auch bei temporären Nulldurchgängen der Gradientenfelder in der Ebene des Implantats zu gewährleisten. Dadurch wird sichergestellt, dass selbst bei ungünstigen Positionen des Implantats gegenüber den Gradientenfeldern eine Detektion der Gradientenfelder möglich oder zumindest sehr wahrscheinlich ist, da sich die Position der Gradientenfelder in der Regel zeitlich verändert um verschiedene Bereiche bei einer MRI-Untersuchung visualisieren zu können. Unter VF (ventricular fibrillation) sind in diesem Zusammenhang nicht nur ventrikuläre Fibrillationen zu verstehen, sondern alle schnellen oder zu schnellen Herzrhythmusstörungen, wobei die Begrifflichkeit zu schnell für jeden Patienten individuell verschieden sein kann, je nach dem Zustand des Herzkreislaufsystems. Explizit eingeschlossen in VF sind sämtlichen ventrikulären Tachykardien.

Eine weitere bevorzugte Ausführungsform ist, dass die Verlängerung der VF-Detektion um die zweite Zeit bis zu 30 s beträgt, bevorzugt bis 10 s beträgt.

Auch wird bevorzugt, dass die Erkennungseinheit für MRT-Störfelder (250) mit mindestens einem weiteren MRT-Sensor und/oder Indikator für MRT-Störfelder verbindbar ist und die MRT-Erkennung auf der Erkennung durch mindestens einen der Sensoren und/oder Indikatoren beruht. Unter MRT-Sensor oder MRT-Indikator sind im Zusammenhang mit dieser Anmeldung alle Sensoren oder Geräte oder Bauteile zu verstehen, die eine Erkennung von MRT-Feldern oder anderen starken elektromagnetischen Feldern ermöglichen. Dazu gehören unter anderem, aber nicht beschränkt auf diese, GMR-Sensoren, Mag-FET-Sensoren, Hallsensoren, die Überwachung von Batteriespannungen während Kondensatorladeprozessen, die Detektion von RF-Feldern, die Detektion von durch elektromagnetische Felder induzierten Strömen, die Detektion durch Leuchtdioden, die von MRT-Feldern zur Lichtemission angeregt werden, die Detektion von spezifischen Vibrationen oder als Sensoren ausgelegte Bauteile zur Detektion von durch Lorentzkräfte induzierten Vibrationen. Des Weiteren kann auch ein Lagesensor, speziell ein selbstkalibrierender Lagesensor, zur Erhöhung der Spezifität der MRT-Erkennung herangezogen werden.

Des Weiteren wird bevorzugt, dass ein Lagesensor zur Plausibilitätsprüfung herangezogen wird und eine positive MRI-Erkennung nur erfolgt, wenn der Lagesensor eine liegende Positur und/oder eine andere voreinstellbare Positur meldet.

Besonders bevorzugt ist der Lagesensor selbst kalibrierend, wobei die Kalibrierung unter voreinstellbaren Randbedingungen, wie aber nicht beschränkt auf, Uhrzeiten und/oder Herzraten und/oder Atemfrequenz und/oder hämodynamische Parameter und/oder Aktivität (Bewegungssensor) stattfindet.

Ebenfalls bevorzugt wird, dass mindestens eine der folgenden Maßnahmen bei einer MRT-Erkennung oder durch das MRT-Erkennungssignal eingeleitet wird:
das Wechseln in einen MRI-Sicherenzustand,
ein verlängertes Verbleiben in einem MRI-sicheren oder gegenüber elektromagnetischen Störfeldern unempfindlichen Zustand,
eine Synchronisierung von elektrischen Messungen (z. B. Impedanzmessungen) mit bei periodischen oder gepulsten elektromagnetischen Feldern auftretenden betragsmäßigen Feldstärkenminima oder die Synchronisierung einer Stimulation mit eben jenen betragsmäßigen Minima und
die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit neben der Störung auch Informationen auf diese Weise zu übertragen und auf dem Bildschirm des MRI sichtbar zu machen.

Die Aufgabe wird auch durch das Verfahren nach Anspruch 11 gelöst. Das Verfahren zur Erkennung von gepulsten magnetischen Wechselfeldern mit einem IMD beinhaltet, dass das IMD durch gepulste elektromagnetische Wechselfelder induzierten Spannungen in der Programmierspule detektiert und in Abhängigkeit von den detektierten induzierten Spannungsverläufen ein Signal im IMD generiert und an eine Steuereinheit im IMD weitergeleitet wird, sofern nicht zeitgleich eine Kommunikation mit einem Programmiergerät über die Programmierspule erkannt wird.

Das Verfahren ist insbesondere mit einem IMD der Ansprüche 1 bis 10 durchführbar.
- Fig. 1: Schematische Darstellung des Ablaufs einer MRT-Untersuchung
- Fig. 2: Blockschaltbild eines erfindungsgemäßen IMD mit einer Erkennungseinheit für MRT-Störfelder
- Fig. 3: Blockschaltbild eines erfindungsgemäßen IMD mit einem einfachen Gradientenfelddetektor
- Fig. 4: Blockschaltbild eines erfindungsgemäßen IMD mit einem spezifisch programmierbaren Gradientenfelddetektor

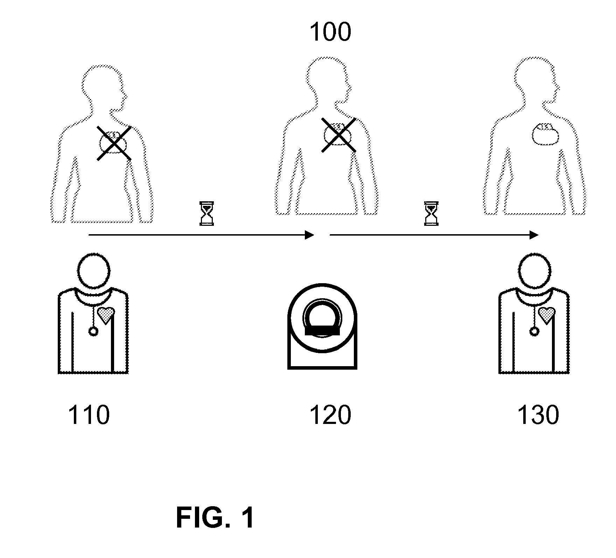

In der Figur 1 ist die Ausgangssituation dargestellt. Der ICD-Patient (100) wird vor der geplanten MRT-Untersuchung von einem Kardiologen nachgesorgt und der ICD ausgeschaltet (110). Mit einer zeitlichen Verzögerung von Stunden bis Tagen erfolgt die MRT-Untersuchung bei einem Radiologen (120). Nach einer weiteren Verzögerung wird der Patient wieder vom Kardiologen (130) betreut und der ICD wieder eingeschaltet. Während der gesamten Zeit von (110) bis (130) ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung in Kauf genommen. Ebenso ist der finanzielle und logistische Aufwand einer solchen Prozedur sehr hoch und schließt in vielen Fällen die Notfallanwendung eines MRT aus.

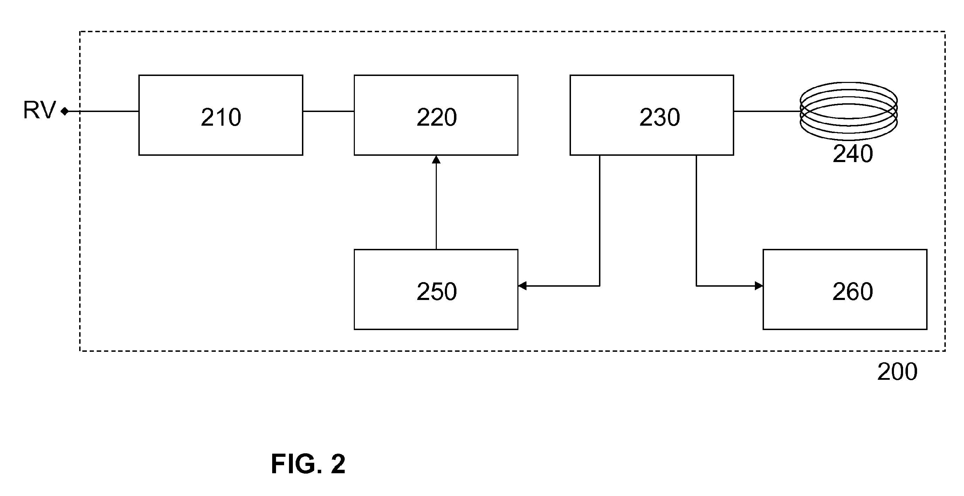

Die Figur 2 zeigt das Blockschaltbild der erfindungsgemäßen Lösung zur Detektion des MRT-Störfeldes im elektronischen Implantat (200). Als Beispiel ist für eine einfache Darstellung ein Einkammer-Schrittmacher ausgewählt, es wird aber darauf verwiesen, dass auch reine Überwachungsgeräte oder Mehrkammergeräte, wie Zwei- oder Drei- oder Vierkammersysteme oder andere Neurostimulatoren herangezogen werden können. Die rechtsventrikuläre Schrittmacherelektrode (RV) ist mit einer Sensingstufe (210) verbunden. Die Sensingstufe (210) ist mit dem Schrittmacherzeitgeber (220) verbunden. Dieser Zeitgeber (220) arbeitet üblicherweise in einem bedarfsgesteuerten Mode (z. B. VVI-Mode).

Zur Kommunikation zwischen dem elektronischen Implantat und einem externer Programmiergerät ist üblicherweise eine vom metallischen Gehäuse des Implantates umschlossene Programmierspule (240) vorhanden. Die Kommunikation erfolgt mittels magnetischer Wechselfelder (Nahfeldtelemetrie). Hierzu ist diese Programmierspule (240) mit einer Sende- und Empfangseinheit (230) verbunden, die ihrerseits mit einer Einheit zum Lesen und Schreiben des Speichers im Implantat (260) verbunden ist. So ist es möglich, dass sowohl Daten aus dem Implantat ausgelesen als auch Parameter in den Speicher des Implantates geschrieben werden können.

Erfindungsgemäß ist zusätzlich die Sende- und Empfangseinheit mit einer Erkennungseinheit für MRT-Störfelder (250) verbunden. Diese wertet die typischen Störmuster eines MRT-Gradientenfeldes, eingekoppelt auf die Programmierspule (240), aus. Die Erkennungseinheit für MRT-Störfelder (250) ist wiederum mit dem Schrittmacherzeitgeber (220) verbunden. Wird ein MRT-Gradientenfeld im Umfeld des elektronischen Implantates wahrgenommen, so wird der Schrittmacherzeitgeber (220) von der Erkennungseinheit für MRT-Störfelder (250) zur automatischen Umschaltung in eine als MRT-sicher definierte Betriebsart umgeschaltet (z. B. V00-Mode für schrittmacherabhängige Patienten).

Alternative Beispiele sind, wie oben erwähnt, die Implementierung in einem ICD (implanted Cardioverter-Defibrillator), Neurostimulator, CRT-Gerät (Cardiac Resynchronization Therapy-Gerät), ein überwachendes Implantat oder eine implantierbaren Medikamentenpumpe.

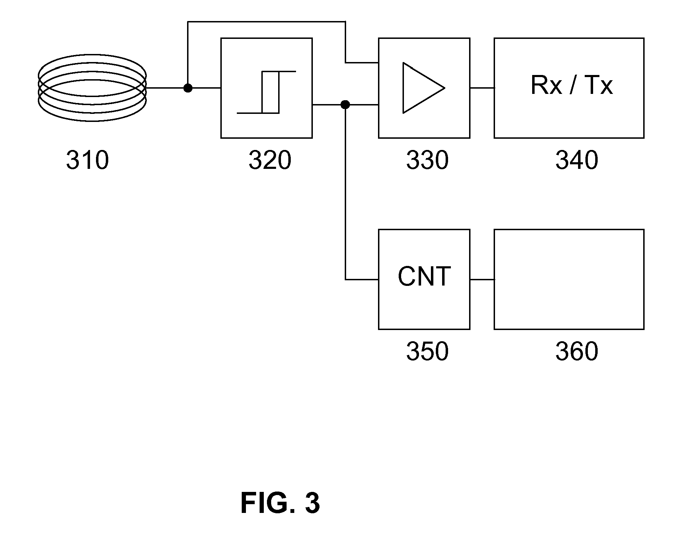

Die Figur 3 zeigt eine einfache Realisierung der Erkennungseinheit für MRT-Störfelder oder auch Gradientenerkennungseinheit, die so in bereits vorhandenen Schrittmacher- und ICD-Systemen "nachrüstbar" wäre. Die Signale der Programmierspule (310) werden der Sende- und Empfangseinheit (340) üblicherweise als verstärkte Signale zugeführt. Allerdings verbraucht dieser sog. Programmierverstärker (330) einen erheblichen Strom, so dass dieser üblicherweise abgeschaltet ist und immer nur dann aktiviert wird, wenn eine zusätzliche, unspezifische Triggereinheit (320) einen Signalpegel an der Programmierspule (310) wahrnimmt. Diese Triggereinheit (320) aktiviert dann den Programmierverstärker (330) und startet so die Auswertung der Programmiersignale in der Sende- und Empfangseinheit (340).

Erfindungsgemäß wird zusätzlich das Ausgangssignal der Triggereinheit (320) einer Zählereinheit (350) zugeführt. Diese Zählereinheit (350) ist dabei so konfiguriert, dass diese immer dann ein Ausgangssignal erzeugt, wenn die Eingangssignalfrequenz einem typischen MRT-Gradientenfeld entsprechen könnte und somit den Verdacht eines MRT-Gradientenfeldes signalisiert. Um diesen "Verdacht" von einer Programmerkommunikation zu differenzieren, ist die Zählereinheit (350) mit einer zusätzlichen Auswerteeinheit verbunden, die nur dann eine Umschaltung in einen MRT-sicheren Mode veranlasst, wenn nicht gleichzeitig eine Programmerkommunikation läuft. Diese Einheit kann außerdem zusätzliche Kriterien, wie z. B. das gleichzeitige Einsetzen von Störungen (Noise) am Elektrodeninterface, hervorgerufen durch die MRT-typische RF-Felder, zur Differenzierung heranziehen.

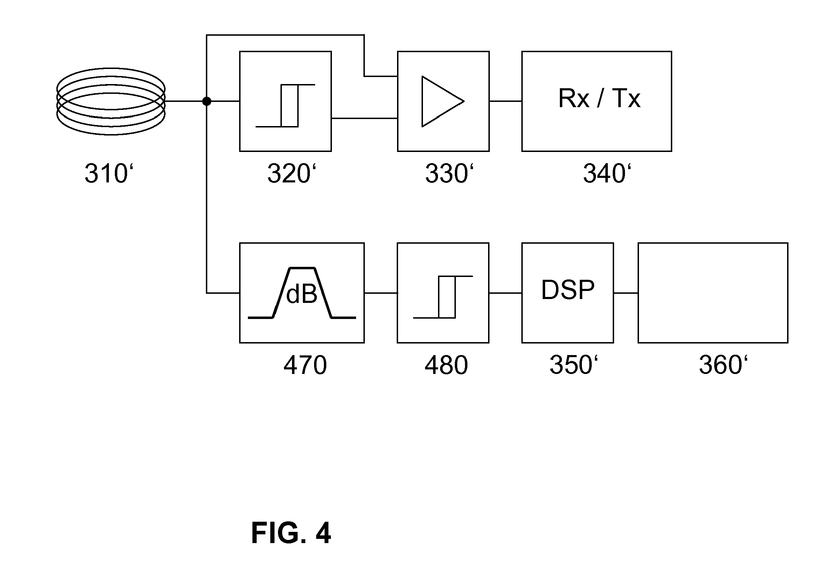

Die Figur 4 zeigt die Erweiterung der in Figur 3 beschriebenen Erkennungseinheit für MRT-Störfelder, insbesondere Gradientenfelder. Hier wird das Signal der Programmierspule (310') für die Gradientenfelddetektion zunächst mittels eines programmierbaren Filters (470) aus das typisch zu erwartende MRT-Gradientenfeld gefiltert, so dass die anschließende separate, programmierbare Triggereinheit (480) nur dann ein Signal liefert, wenn eine Störung im Frequenzspektrum eines MRT-Gradientenfeldes an der Programmierspule (320') auftritt.
Zusätzlich kann an Stelle der o. g. Zählereinheit auch eine algorithmische Bewertung der Gradientensignale z. B. in einem digitalen Signalprozessor (350') durchgeführt werden, um so die typischen Wiederholraten und Muster eines MRT-Gradientenfeldes sicher von anderen Störsignalen unterscheiden zu können.
Dies hat den Vorteil, dass die Erkennungseinheit für MRT-Störfelder oder der Gradientenfelddetektor spezifisch an definierte MRT-Systeme angepasst werden kann und das Risiko einer versehentlichen Umschaltung ein einen MRT-sicheren Zustand minimiert wird, was die Spezifität der Erkennungseinheit für MRT-Störfelder.

## Patentansprüche

1. Implantierbares medizinisches Gerät (IMD) (200) mit einem hermetisch abgeschlossenem Gehäuse, mindestens einer Steuereinheit, mindestens einer Programmierspule (240), mindestens einer Kommunikationseinheit und einer Erkennungseinheit für MRT-Störfelder (250), wobei die Kommunikationseinheit im Zusammenwirken mit der Programmierspule (240) ausgestaltet ist, eine Kommunikation zwischen einem externen Programmiergerät und dem implantierten medizinischen Gerät (200) unter Nutzung von elektromagnetischen Wechselfeldern zu ermöglichen, **gekennzeichnet dadurch, dass**
die Erkennungseinheit für MRT-Störfelder (250) derart ausgebildet ist, dass von einem gepulsten elektromagnetischen Wechselfeld des MRT herrührende in die Programmierspule (240) induzierte Spannungen oder Spannungsverläufe erkannt werden, und ferner derart ausgebildet ist, dass ein MRT-Erkennungssignal von der Erkennungseinheit für MRT-Störfelder (250) an die mindestens eine Steuereinheit übermittelt wird, sofern nicht zeitgleich eine Kommunikation mit einem externen Programmiergerät über die Programmierspule erkannt wird.

2. IMD nach Anspruch 1, **dadurch gekennzeichnet, dass** die Erkennungseinheit für MRT-Störfelder (250) die gepulsten elektromagnetischen Wechselfelder durch eine Schwellwertdetektion und eine Zählung der induzierten Spannungspulse pro Zeiteinheit erkennt.

3. IMD nach einem der vorangegangenen Ansprüche, **dadurch gekennzeichnet, dass** die MRT-Störerkennung auf Auswertung und Vergleich der induzierten Spannungen oder des Spektrums der induzierten Spannungen mit MRT-typischen induzierten Spannungen oder MRT-typischen induzierten Spannungsspektren beruht.

4. IMD nach einem der vorangegangenen Ansprüche, **dadurch gekennzeichnet, dass** das IMD zusätzlich mit mindestens einer Elektrode verbunden ist, die mit der Erkennungseinheit für MRT-Störfelder (250) verbunden ist und ein MRT-Erkennungssignal nur dann übermittelt wird, wenn die Störung zeitgleich sowohl über die Programmierspule (240), als auch über mindestens eine Elektrode erkannt wird.

5. IMD nach einem der vorangegangenen Ansprüche, **dadurch gekennzeichnet, dass** das MRT-Erkennungssignal einen Wechsel des Betriebszustands des IMD bewirkt.

6. IMD nach einem der vorangegangenen Ansprüche, **dadurch gekennzeichnet, dass** die Erkennung der gepulsten elektromagnetischen Wechselfelder über ein programmierbares Filter und eine Triggereinheit erfolgt, die mit der Programmierspule (240) verbunden sind.

7. IMD nach einem der vorangegangenen Ansprüche, **dadurch gekennzeichnet, dass** ein MRT-Zustand permanent, bis zur nächsten Umprogrammierung oder für eine vorbestimmte oder eine einstellbare erste Zeit einstellbar ist, und eine VF-Detektion um eine vorbestimmte zweite Zeit verlängert wird, um eine Erkennung der gepulsten elektromagnetischen Wechselfeldern auch bei temporären Nulldurchgängen der Gradientenfelder in der Ebene des Implantats zu gewährleisten.

8. IMD nach Anspruch 7, **dadurch gekennzeichnet, dass** die Verlängerung der VF-Detektion um die zweite Zeit bis zu 30 s beträgt, bevorzugt bis 10 s beträgt.

9. IMD nach einem der vorangegangenen Ansprüche, **dadurch gekennzeichnet, dass** die Erkennungseinheit für MRT-Störfelder (250) mit mindestens einem weiteren MRT-Sensor und/oder Indikator für MRT-Störfelder verbindbar ist und die MRT-Erkennung auf der Erkennung durch mindestens einen der Sensoren und/oder Indikatoren beruht.

10. IMD nach einem der vorangegangenen Ansprüche, **dadurch gekennzeichnet, dass** mindestens eine der folgenden Maßnahmen bei einer MRT-Erkennung eingeleitet werden:
- das Wechseln in einen MRI-sicheren Zustand
- ein verlängertes Verbleiben in einem MRI-sicheren oder gegenüber elektromagnetischen Störfeldern unempfindlichen Zustand,
- eine Synchronisierung von elektrischen Messungen (z. B. Impedanzmessungen) mit bei periodischen oder gepulsten elektromagnetischen Feldern auftretenden betragsmäßigen Feldstärkenminima oder die Synchronisierung einer Stimulation mit eben jenen betragsmäßigen Minima, und
- die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit neben der Störung auch Informationen auf diese Weise zu übertragen und auf dem Bildschirm des MRI sichtbar zu machen.

11. Verfahren zur Erkennung von gepulsten magnetischen Wechselfeldern mit einem IMD, **dadurch gekennzeichnet, dass** das IMD durch gepulste elektromagnetische Wechselfelder induzierten Spannungen in der Programmierspule detektiert und in Abhängigkeit von den detektierten induzierten Spannungsverläufen ein Signal im IMD generiert, ferner **dadurch gekennzeichnet, dass** das Signal an eine Steuereinheit im IMD weitergeleitet wird, sofern nicht zeitgleich eine Kommunikation mit einem Programmiergerät über die Programmierspule erkannt wird.

## Claims

1. An implantable medical device (IMD) (200), comprising a hermetically sealed housing, at least one control unit, at least one programming coil (240), at least one communication unit, and a detection unit for MRT interference (250), the communication unit being designed in cooperation with the programming coil (240) to enable communication between an external programming device and the implantable medical device (200) using alternating electromagnetic fields, **characterized in that**
the detection unit for MRT interference (250) is designed in such a way that voltages or voltage curves induced in the programming coil (240) and originating from a pulsed alternating electromagnetic field of the MRT are detected and is further designed in such a way that an MRT detection signal is transmitted from the detection unit for MRT interference (250) to the at least one control unit, unless communication with an external programming device via the programming coil is detected at the same time.

2. The IMD according to claim 1, **characterized in that** the detection unit for MRT interference (250) detects the pulsed alternating electromagnetic fields by way of threshold value detection and counting of the induced voltage pulses per unit of time.

3. An IMD according to any one of the preceding claims, **characterized in that** the MRT interference detection is based on an evaluation and comparison of the induced voltages, or of the spectrum of the induced voltages, with MRT-typical induced voltages, or MRT-typical induced voltage spectra.

4. An IMD according to any one of the preceding claims, **characterized in that** the IMD is additionally connected to at least one electrode, which is connected to the detection unit for MRT interference (250), and an MRT detection signal is only transmitted when the interference is detected simultaneously both via the programming coil (240) and via at least one electrode.

5. An IMD according to any one of the preceding claims, **characterized in that** the MRT detection signal causes a change in the operating state of the IMD.

6. An IMD according to any one of the preceding claims, **characterized in that** the detection of the pulsed alternating electromagnetic fields is carried out by way of a programmable filter and a trigger unit, which are connected to the programming coil (240).

7. An IMD according to any one of the preceding claims, **characterized in that** an MRT state can be set permanently, until the next reprogramming, or for a predetermined or a settable first time, and a VF detection is extended by a predetermined second time so as to ensure a detection of the pulsed alternating electromagnetic fields even with temporary zero crossings of the gradient fields in the plane of the implant.

8. The IMD according to claim 7, **characterized in that** the extension of the VF detection by the second time is up to 30 s, and preferably up to 10 s.

9. An IMD according to any one of the preceding claims, **characterized in that** the detection unit for MRT interference (250) can be connected to at least one further MRT sensor and/or indicator for MRT interference, and the MRT detection is based on the detection by at least one of the sensors and/or indicators.

10. An IMD according to any one of the preceding claims, **characterized in that** at least one of the following measures is initiated during an MRT detection:
- switching to an MRI-safe state;
- extended remaining in an MRI-safe state or a state that is not sensitive to electromagnetic interference;
- synchronizing electric measurements (such as impedance measurements) with absolute field strength minima occurring with periodic or pulsed electromagnetic fields, or synchronizing a stimulation with these very absolute minima; and
- delivering electromagnetic pulses to indicate that a medical device, specifically an implant, is present in the electromagnetic field, specifically to indicate this to an MRI device, with the option to also transmit information in this way, in addition to the interference, and render this information visible on the screen of the MRI.

11. A method for detecting pulsed alternating magnetic field using an IMD, **characterized in that** the IMD detects voltages in the programming coil induced by pulsed alternating electromagnetic fields and generates a signal in the IMD as a function of the detected induced voltage curves, further **characterized in that** the signal is forwarded to a control unit in the IMD, unless communication with a programming device via the programming coil is detected at the same time.

## Revendications

1. Appareil médical implantable (IMD) (200) avec un boîtier fermé hermétiquement, au moins une unité de commande, au moins une bobine de programmation (240), au moins une unité de communication et une unité de reconnaissance de champs parasites d'IRM (250), dans lequel l'unité de communication, en coopération avec la bobine de programmation (240), est conçue pour permettre une communication entre un appareil de programmation externe et l'appareil médical implantable (200), à l'aide de champs alternatifs électromagnétiques, **caractérisé en ce que**
l'unité de reconnaissance de champs parasites d'IRM (250) est conçue de manière à ce que des tensions ou des courbes de tension induites dans la bobine de programmation (240), provoquées par un champ alternatif électromagnétique pulsé de l'IRM soient reconnues, et conçue en outre de manière à ce que un signal de reconnaissance IRM soit transmis de l'unité de reconnaissance de champs parasites d'IRM (250) à l'au moins une unité de commande, dans la mesure où une communication avec un appareil de programmation externe n'est pas simultanément reconnue par le biais de la bobine de programmation.

2. IMD selon la revendication 1, **caractérisé en ce que** l'unité de reconnaissance de champs parasites d'IRM (250) reconnaît les champs alternatifs électromagnétiques pulsés par une détection de valeur seuil et un comptage des impulsions de tension induites par unité de temps.

3. IMD selon l'une des revendications précédentes, **caractérisé en ce que** la reconnaissance de parasites d'IRM repose sur une évaluation et une comparaison des tensions induites ou du spectre des tensions induites avec des tensions induites typiques d'IRM ou des spectres de tensions induites typiques d'IRM.

4. IMD selon l'une des revendications précédentes, **caractérisé en ce que** l'IMD est en outre relié à une électrode, laquelle est reliée à l'unité de reconnaissance de champs parasites d'IRM (250), et **en ce qu'**un signal de reconnaissance d'IRM n'est transmis que lorsque la perturbation est reconnue simultanément par le biais de la bobine de programmation (240) et par le biais d'au moins une électrode.

5. IMD selon l'une des revendications précédentes, **caractérisé en ce que** le signal de reconnaissance d'IRM produit un changement de l'état de fonctionnement.

6. IMD selon l'une des revendications précédentes, **caractérisé en ce que** la reconnaissance des champs alternatifs électromagnétiques pulsés est effectuée par le biais d'un filtre programmable et d'une unité de déclenchement reliés à la bobine de programmation (240).

7. IMD selon l'une des revendications précédentes, **caractérisé en ce qu'**un état d'IRM peut être réglé permanent, jusqu'à la prochaine reprogrammation ou pour une durée prédéfinie ou réglable, et une détection VF est prolongée d'un deuxième temps prédéfini, pour garantir également une reconnaissance des champs alternatifs électromagnétiques pulsés lors de passages par zéro temporaires des champs de gradients dans le plan de l'implant.

8. IMD selon la revendication 7, **caractérisé en ce que** le prolongement de la détection VF sur le deuxième temps dure jusqu'à 30 secondes, de préférence jusqu'à 10 secondes.

9. IMD selon l'une des revendications précédentes, **caractérisé en ce que** l'unité de reconnaissance de champs parasites d'IRM (250) peut être reliée à au moins un autre capteur d'IRM et/ou indicateur de champs parasites d'IRM, et la reconnaissance d'IRM repose sur la reconnaissance par au moins un capteur et/ou indicateur.

10. IMD selon l'une des revendications précédentes, **caractérisé en ce qu'**au moins l'une des mesures suivantes est introduite lors d'une reconnaissance d'IRM :
- le passage à un état d'IRM sécurisé,
- la prolongation d'un état d'IRM sécurisé ou insensible aux champs parasites électromagnétiques,
- une synchronisation de mesures électriques (par exemple des mesures d'impédance) avec des montants minimums d'intensités de champs apparaissant avec des champs électromagnétiques périodiques ou pulsés, ou la synchronisation d'une stimulation avec ces montants minimums, et
- la délivrance d'impulsions électromagnétiques pour signaler la présence d'un appareil médical, en particulier d'un implant, dans le champ électromagnétique, en particulier pour le signaler à un appareil d'IRM, avec la possibilité de transmettre également des informations de cette manière, à côté de la perturbation, et de les faire apparaître sur l'écran de l'IRM.

11. Procédé pour la reconnaissance de champs magnétiques alternatifs pulsés avec un IRM, **caractérisé en ce que** l'IRM détecte des tensions induites par des champs alternatifs électromagnétiques pulsés dans la bobine de programmation, et génère un signal dans l'IRM en fonction des courbes de tensions induites détectées, en outre **caractérisé en ce que** le signal est transmis à une unité de communication dans l'IRM, dans la mesure où une communication avec un appareil de programmation n'est pas reconnue simultanément par le biais de la bobine de programmation.
